# EUROPEAN PATENT SPECIFICATION

(11) **EP 0 587 912 B1**
(45) Date of publication and mention of the grant of the patent: **10.09.1997**
(21) Application number: 93906866.4
(22) Date of filing: 01.04.1993
(51) Int. Cl.: F04B 43/04

(54) **FLUID CONTROLLING MICRODEVICE AND METHOD OF MANUFACTURING THE SAME**
MIKROSTEUERVORRICHTUNG FUER FLUIDUM UND VERFAHREN ZU DEREN HERSTELLUNG
MICRODISPOSITIF DE REGULATION DE DEBIT DE FLUIDE, ET PROCEDE DE FABRICATION DUDIT DISPOSITIF

(30) Priority: 02.04.1992 JP 81040/92; 24.04.1992 JP 106887/92; 24.04.1992 JP 106888/92
(43) Date of publication of application: 23.03.1994
(73) Proprietor: SEIKO EPSON CORPORATION, Shinjuku-ku Tokyo 163-08 (JP)
(72) Inventor: MIYAZAKI, Hajime, Suwa-shi, Nagano 392 (JP); NOSE, Yasuto, Suwa-shi, Nagano 392 (JP); ARAKAWA, Katsuji, Suwa-shi, Nagano 392 (JP); SHIMIZU, Nobuo, Suwa-shi, Nagano 392 (JP); MURANAKA, Tsukasa, Suwa-shi, Nagano 392 (JP)
(74) Representative: Watkins, David
(86) International application number: JP9300413
(87) International publication number: WO9320351

(56) References cited:
- JP-T- 3 505 771
- JP-T- 4 501 449
- JP-T- 4 502 796
- JP-U-54 040 504
- JP-U-58 004 781

## Description

### TECHNICAL FIELD

The present invention relates to a fluid control micro device such as a micro pump, a micro valve, or the like, and particularly relates to a method for producing an intermediate substrate which is one of main parts thereof.

### BACKGROUND TECHNIQUE

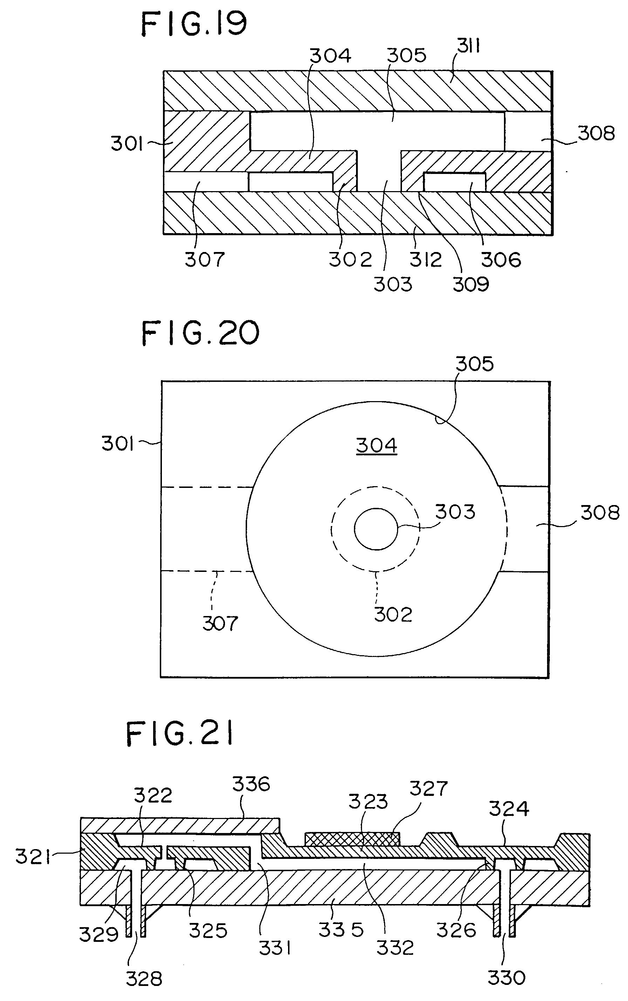

With respect to fluid control micro devices using a thin-film forming technique, a special article entitled "Silicon Micro Machining Technique" was reported in Nikkei Electronics No. 480̸ (published on August 21, 1989) P.125-155. According to the special article, a fluid control micro device is formed by the steps of: forming a valve thin film on a silicon substrate by anisotropic etching; and connecting the silicon substrate with upper and lower glass substrates like a sandwich by anodic bonding. The configuration of the micro device is shown in Fig. 19. Fig. 19 is a sectional side view of the micro device; and Fig. 20 is a top view showing the case where the upper substrate in Fig. 19 is omitted. In the drawings, the reference numeral 30̸1 designates an intermediate silicon substrate in which a valve film 30̸4 having a valve portion 30̸2 and a through hole 30̸3 is formed. Further, chambers 30̸5 and 30̸6 each constituted by a circular concave are formed concentrically in the upper and lower of the valve film 30̸4. The lower chamber 30̸6 is communicated with an input passage 30̸7 shaped like a groove. The upper chamber 30̸5 is communicated with an output passage 30̸8 shaped like a groove. The reference numerals 311 and 312 designate an upper glass substrate and a lower glass substrate, respectively, which are anodically bonded with upper and lower surfaces of the silicon substrate 30̸1 having the aforementioned structure. A seal portion of the valve portion 30̸2 is not anodically bonded with the lower glass substrate 312.

A fluid is introduced into the lower chamber 30̸6 through the input passage 30̸7. Because the valve film 30̸4 is bent upward by pressure of the input fluid at this time so that the seal portion 30̸9 of the valve portion 302 is separated from the lower glass substrate 312 to form a fine gap, the fluid passes through the gap, flows from the through hole 30̸3 to the upper chamber 30̸5 and is outputted as predetermined pressure via the output passage 30̸8. On the other hand, because the valve film 30̸4 is bent downward when the output pressure is heightened compared with the input pressure of the fluid, the seal portion 30̸9 of the valve portion 30̸2 comes into close contact with the lower glass substrate 312 so that a backward flow is prevented.

Next, Fig. 21 shows a conventional example in which the aforementioned micro device technique is applied to a pump. This has been disclosed in the specification of U.S. Patent 5,171,132. In the following, this will be described in brief.

An intermediate silicon substrate 321 is constituted by uniting an input valve film 322, a diaphragm portion 323 and an output valve film 324 into one body. The input valve film 322 has an input valve portion 325, and the output valve film 324 has an output valve portion 326. A piezoelectric element 327 as a driving means is fixed to the upper surface of the diaphragm. An input port 328 passes through the lower glass substrate 335 and is communicated with the input passage 329. Similarly, the output port 330̸ passes through the lower glass substrate 335 and is communicated with the inside of the output valve portion 326. An output passage 331 communicated with the inside of the input valve portion 325 is communicated with the output valve portion 326 via a pressure chamber 332 just under the diaphragm portion 323. The reference numeral 336 designates an upper glass substrate.

With respect to the theory of operation of the micro pump, when the piezoelectric element 327 applies a voltage so as to extend in the direction of a plane, the diaphragm portion 323 following this is bent upward. As a result, the internal pressure of the pressure chamber 332 and the output passage 331 is turned to a negative value, so that the input valve portion 325 is opened to suck a fluid from the input port 328. Further, at this time, pressure is applied on the output valve portion 326 in the direction of closing of the output valve portion 326, so that sealing is performed by the seal portion of the valve portion 326. On the contrary, when the piezoelectric element 327 applies a voltage so as to contract in the direction of a plane, the diaphragm portion 323 is bent downward. As a result, the internal pressure of the pressure chamber 332 and the output passage 331 is turned to a positive value, so that the output valve portion 326 is opened to eject the fluid from the output port 330̸. At this time, pressure is applied to the input valve portion 325 in the direction of closing of the input valve portion 325, so that the fluid is not ejected to the input port 328. By repeating the aforementioned operation, an ejecting operation is obtained.

U.S. Patent 5,085,562 discloses a micropump comprising a pumping chamber, an inlet channel communicating with the pumping chamber via an inlet valve and an outlet channel communicating with the pumping chamber via an outlet valve. These features are formed by etching a silicon wafer and then sealing glass wafers to the opposite faces of the silicon. A piezoelectric wafer is used to vary the volume of the pumping chamber by effecting wall flexure. This document is directed in particular to the provision of stop means which limits the amount of wall flexure, thereby ensuring consistent volume delivery by the pump regardless of its age and the applied electric potential.

U.S. Patent 5,224,843 is also concerned with micropumps. In particular, it is concerned with pumps requiring pressure regulation -to ensure consistent output which may be of importance in certain medical applications. The configuration of the micropump disclosed in this document is such that the pumping chamber is an upper chamber situated over the input valve assembly.

U.S. Patent 5,219,278 describes a micropump similar to the micropump of U.S. Patent 5,224,843 wherein the pumping chamber is positioned over the input valve assembly, but is distinguished therefrom by offsetting the inlet into the pumping chamber so that the internal walls of the pumping chamber are swept by incoming liquid in a controlled manner.

None of the prior art devices is entirely satisfactory.

A fluid control micro device and a method for its production with the features of the preamble of claims 1 and 23 is known from US-A-5 171 132.

The following four points can be described mainly as problems on the conventional fluid control micro device having the aforementioned structures.

The first point is that excretion of bubbles is not easy at the time of initial fluid injection. This is because the presence of bubbles exerts a large influence directly on valve characteristic so that desired valve characteristic cannot be obtained. Particularly, bubbles is apt to remain in the chamber on the input valve portion. When a pressure increasing/pressure reducing operation is carried out in the case where such bubbles remain in the chamber or in the output passage, bubbles are contracted/expanded but the fluid cannot come in from the input port and cannot go out from the output port so that discharge characteristic is lowered rapidly. Although bubbles remain when the inside of the device is filled with the fluid, the main cause of remaining of bubbles is that the depth or thickness of the output side chamber or passage becomes very larger than the depth or thickness of the input side chamber or passage because of limitation on production so that the rate of flow sufficient to discharge bubbles cannot be obtained.

The second point is that entering of dust, contamination, etc. cannot be prevented. This is because when dust, contamination, etc. is trapped between the valve seal portion and the lower substrate, a slight gap is produced in this portion so that a backward flow cannot be prevented. Discharge flow rate characteristic of the micro pump is also deteriorated. Further, when trapping of dust, etc. occurs once, dust, etc. is apt to be stuck to the seal portion so that it becomes more difficult to eject dust, etc.

The third point is that valve sticking cannot be prevented. Because not only the surface of a thermally oxidized film (silicon dioxide) in the valve seal surface generally has surface roughness of average 2 nm which is substantially equal to the surface roughness of the polished surface of a silicon material but the thermally oxidized film and the glass substrate opposite to the thermally oxidized film are of the same kind (silicon oxide) from the viewpoint of the kind of material, the bubble sticking phenomenon occurs so that it becomes difficult to inject the liquid. Furthermore, in the extreme case, injection pressure is increased so that the valve film may be often destroyed.

The fourth point is that entering of bubbles cannot be prevented. When a pressure increasing/pressure reducing operation is carried out in the case where bubbles remaining in an external tank, tube, etc. enters into the pump which is operating, bubbles are contracted/expanded but the fluid cannot come in from the input port and cannot go out from the output port so that discharge characteristic is lowered rapidly. The present invention is to solve the aforementioned problems in the prior art and a first object thereof is to make escaping of bubbles good.

A second object of the invention is to provide a structure in which dust, contamination, etc. cannot enter.

A third object of the invention is to provide a structure in which valve bubble sticking cannot occur.

A fourth object of the invention is to provide a structure in which bubbles cannot enter from the outside.

Other objects of the present invention will become clear in the following description of embodiments.

### DISCLOSURE OF THE INVENTION

According to the invention which is claimed in claim 1, there is provided a fluid control micro device comprising three substrates joined in a multi-layer structure by an anodic joining method, said three substrates being an upper substrate, an intermediate substrate and a lower substrate, said intermediate substrate comprising a valve film which separates an input side space formed between said upper and lower substrates into an upper chamber and a lower chamber, said valve film having a cylindrical valve portion for communicating or blocking said upper chamber and said lower chamber in accordance with upward and downward transformation of said valve film, wherein the ratio of the thickness t2 of said upper chamber to the thickness tl of said lower chamber is selected to be in the range t2/tl = 0.2 to 5.0, characterized in that said upper substrate and said intermediate substrate are joined at a step portion to reduce the thickness of said upper chamber.

In the configuration of the present invention, the difference between the thicknesses of the two chambers is reduced to thereby increase the flow rate of the output passage at the time of initial liquid injection, so that bubbles which are apt to remain in the upper chamber can be ejected out with the high flow rate. As a result, bubbles do not remain, so that desired valve characteristic can be maintained. The reason why 0̸.2 ≦ t2/t1 is selected is that the variable range of the valve film is limited extremely so that the valve film cannot fulfill the valve function per se if the ratio is smaller than this value. The reason why t2/t1≦5.0̸ is selected is that bubbles are apt to remain in the same manner as in the conventional case if the ratio is larger than this value.

Further, the present invention is practically configured so that the upper chamber or lower chamber has a step portion joined with a corresponding supporting substrate so as to satisfy the thickness range. Because it is impossible to thin the intermediate substrate so sufficiently, the aforementioned thickness ratio is maintained by providing the step portion. Further, the upper chamber can be formed on the upper substrate side or can be formed both on the upper substrate and on the intermediate substrate, in accordance with the position of height of the step portion from the valve film.

Also in the case of a micro pump, the upper and lower chambers on the input valve portion side are formed so as to satisfy the aforementioned thickness ratio.

Preferably, the fluid control micro device of the present invention is further characterized in that said intermediate substrate has a pressure chamber communicating with an output passage of a flow passage system, a diaphragm portion above said pressure chamber, a piezo-electric element for driving said diaphragm portion, and an output valve portion communicating with said pressure chamber for opening/closing an output port, said fluid control micro device constituting a micro pump.

In a variant of the present fluid control micro device, the flow passage system in said intermediate substrate is located adjacent the upper substrate.

Conveniently, the fluid control micro device is further characterized in that a filter portion constituted by at least one groove is provided in the input passage in the flow passage system. Because dust, contamination, etc. mixed in the input fluid is removed by the filter portion, there is no risk that dust, etc. is interposed on the seal surface of the valve portion.

The filter portion may be formed in the intermediate substrate so as to be shaped like a comb or may be formed in the supporting substrate opposite to the valve portion so as to be shaped like a comb. Alternatively, a narrow gap may be provided between the projecting portion provided in the intermediate substrate and the supporting substrate opposite to the valve portion so that the narrow portion can be used as a filter portion.

In a further embodiment of the present invention, a hydrophilic filter is attached directly to a portion of an input port provided in the supporting substrate. Since bubbles from outside are prevented from entering by said filter portion, said bubbles cannot enter the inside of pump. Said hydrophilic filter may be combined with the laminate of a plurality of filters different in pore size as construction in the embodiment of the present invention. When a laminate of a plurality of filters different in pore size and a hydrophilic filter are attached to a portion of the input port of the micro pump, not only entering of bubbles but entering of dust, contamination, etc. can be inhibited simultaneously. The "hydrophilic filter" means a filter which is pervious to water but impervious to air.

Further, both the structure to satisfy the aforementioned chamber thickness ratio and the filter structure can be provided simultaneously.

In a still further embodiment of the present invention, at least one of the seal surface of the valve portion and the surface portion of the supporting substrate opposite to the valve portion is formed as a rough surface having a surface roughness range from 5 nm to 1000 nm as average.

When the valve seal surface or the surface portion of the supporting substrate opposite to the valve portion is formed as a rough surface as described above, the liquid penetrates into the clearance of the rough surface easily so that the valve portion is separated from the supporting substrate by low pressure. Accordingly, there arises an effect that bubble sticking does not occur. Further, there is no occurrence of liquid leakage as long as the surface roughness is in the aforementioned range.

The aforementioned rough surface can be formed as a shape in which a plurality of grooves are provided. In this case, the plurality of grooves are formed circumferentially in the valve seal surface or formed concentrically in the surface portion of the supporting substrate.

Further, after the valve seal surface is formed by a pre-load layer of the thermally oxidized film, a platina thin film layer may be further formed on the pre-load layer.

Further, the contact area of the seal portion of the valve portion may be reduced by tapering or rounding the seal portion.

The device of the present invention may have a small-scaled hard tank connected to an input port of the flow passage system, and a large-scaled soft tank connected to the small-scaled hard tank, the small-scaled hard tank having a septum attached to the wall thereof, and a check valve for inhibiting flowing of a fluid into the large-scaled soft tank.

By providing such a small-scaled hard tank between the micro pump and the large-scaled soft tank, liquid can be injected into the small-scaled hard tank while the injection needle is thrust into the septum. Furthermore, the check valve prevents the thus injected fluid from entering into the large-scaled soft tank, so that liquid enters into the micro pump smoothly because the soft tank is not expanded. Accordingly, priming of the micro pump can be performed easily. Further, entering of bubbles, dust, contamination, etc. into the micro pump can be prevented by attaching a laminate of a plurality of filters different in pore size and a hydrophilic filter to a portion of the input port of the flow passage system as described above.

In another variant, the device has a hard tank connected to an input port of the flow passage system, the hard tank having a hydrophilic filter attached to the connection port thereof and a part of the inner surface, and a hydrophobic filter and a septum attached to the wall portion of the hard tank in the case where the hydrophilic filter is not attached to the hard tank. Even in such structure, not only entering of bubbles into the micro pump can be prevented but priming can be performed easily. Furthermore, even if the quantity of the liquid in the hard tank is reduced to a small value, the liquid can be supplied by using the hydrophilic filter as a passage. The "hydrophobic filter" means a filter which has properties reverse to those of the hydrophilic filter and which is impervious to water but pervious to air. The hydrophobic filter fulfills the respiratory function for the hard tank.

In yet another embodiment, the device may have relay pipe members connected to the flow passage system, the relay pipe members having a relay pipe formed to have a shape in which the inner surface of the relay pipe is tapered or rounded so that the diameter of the inner hole of the relay pipe increase to the outer circumferential edge at each of the opposite end portions of the relay pipe.

The configuration characterized by the producing method of the present invention described in claim 23 is in a method for producing the aforementioned intermediate substrate, a step portion equal in height to a diaphragm portion is formed simultaneously on an input side upper chamber by applying anisotropic etching to a silicon substrate by using a pattern mask. The difference between the thickness of the input valve film and the thickness of the diaphragm can be formed directly as the thickness of the upper chamber.

### BRIEF DESCRIPTION OF THE DRAWINGS

Fig. 1 is a sectional side view of a micro device according to a first embodiment of the present invention.

Fig. 2 is a top view showing the case where the upper substrate in the first embodiment is omitted.

Fig. 3 is an explanatory view for explaining bubble escaping in the first embodiment.

Fig. 4 is a sectional side view showing a second embodiment of the present invention.

Fig. 5 is a sectional view taken along the line A-A in Fig. 4.

Fig. 6 is a sectional side view showing a third embodiment of the present invention.

Fig. 7 is a top view showing the case where the upper substrate in the third embodiment is omitted.

Fig. 8 is a sectional side view showing a fourth embodiment of the present invention.

Fig. 9 is a sectional side view showing a fifth embodiment of the present invention and a producing step view of a micro pump.

Fig. 10 is an enlarged sectional view of a valve portion in a sixth embodiment of the present invention.

Fig. 11 is an enlarged sectional view of a valve portion in a seventh embodiment of the present invention.

Fig. 12 is an enlarged sectional view of a valve portion in an eighth embodiment of the present invention.

Fig. 13 is an enlarged sectional view of a valve portion in a ninth embodiment of the present invention.

Fig. 14 is an enlarged sectional view of a valve portion showing a modified example of the ninth embodiment.

Fig. 15 is a schematic structural view in partial section showing a micro pump system according to a tenth embodiment of the present invention.

Fig. 16 is a schematic structural view in partial section showing a micro pump system according to an eleventh embodiment of the present invention.

Fig. 17 is a sectional view of a relay pipe of pipe members according to a twelfth embodiment of the present invention.

Fig. 18 is a schematic producing step view of an intermediate substrate in the present invention.

Fig. 19 is a sectional side view of a conventional fluid control micro device.

Fig. 20 is a top view showing the case where the upper substrate in Fig. 19 is omitted.

Fig. 21 is a sectional view of a conventional micro pump.

### PREFERRED EMBODIMENTS OF THE INVENTION

Embodiments of the present invention will be described below with reference to the drawings.

### EMBODIMENT 1

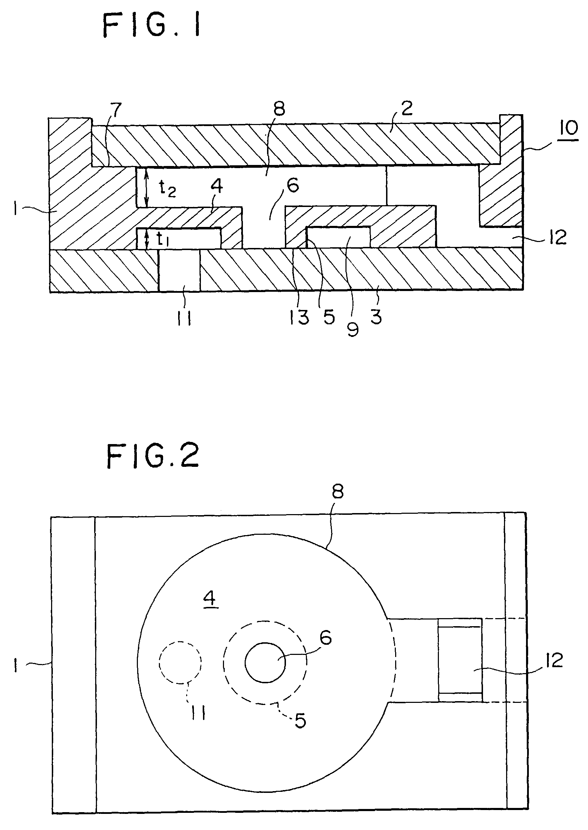

Fig. 1 is a sectional side view of a micro device showing a first embodiment of the present invention; and Fig. 2 is a top view of the micro device showing the case where an upper substrate is omitted.

The micro device 10̸ has a structure in which an intermediate silicon substrate 1 is sandwiched between an upper glass substrate 2 and a lower glass substrate 3.

For example, the board thickness of the intermediate silicon substrate is 50̸0̸ µm after both surfaces thereof are polished. A valve film 4, a valve portion 5 and a vent 6 are formed in the intermediate substrate 1 by anisotropic etching and, at the same time, an output-side upper chamber 8 provided with a step portion 7, an input-side lower chamber 9 and an output passage 12 communicated with the upper chamber 8 are formed. In the intermediate substrate 1, a fluid passage system is constituted by these lower chamber 9, vent 6, upper chamber 8 and output passage 12.

Further, the upper substrate 2 is anodically bonded to the step portion 7 of the intermediate substrate 1 whereas the lower substrate 3 preliminarily provided with an input port 11 is anodically connected to the lower surface of the intermediate substrate 1 so that the input port 11 is communicated with the lower chamber 9. A seal surface 7 of the valve portion 5 is not anodically connected to the lower substrate 3.

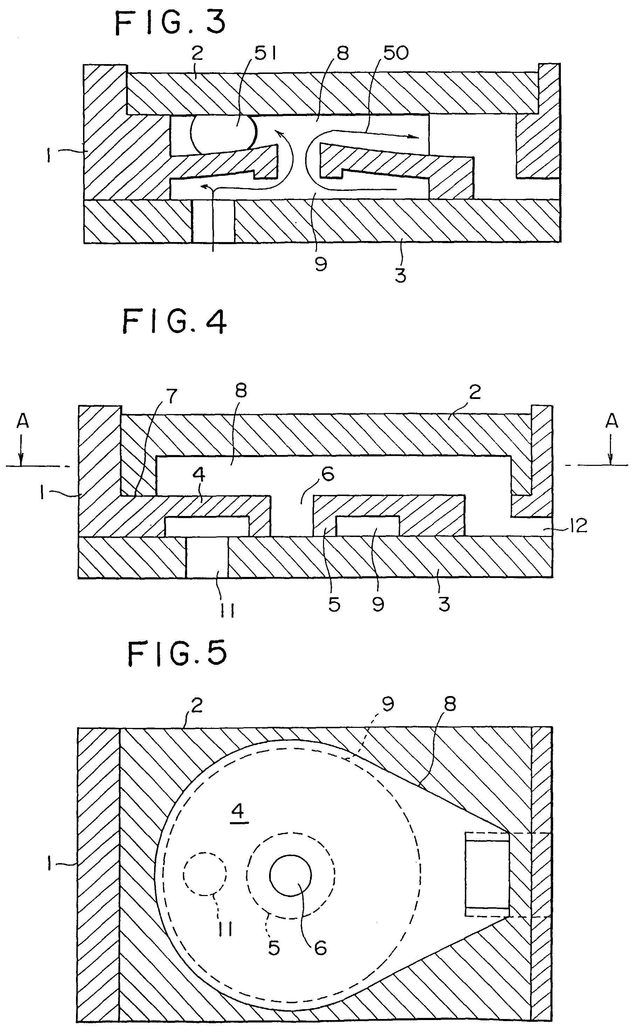

By providing the step portion 7 as described above, the thickness t2 of the upper chamber 8 can be reduced, so that the ratio t2/t1 of the thickness of the upper chamber 8 to the thickness of the lower chamber 9 can be approached substantially to 1.0̸. When, for example, the thickness of the intermediate substrate 1, the diameter of the valve film 4, i.e. the diameter of the upper and lower chambers 8 and 9, the thickness of the lower chamber 9 and the thickness of the valve film 4 are selected to be 50̸0̸ µm, 5 mm, 50̸ µm, and 50̸ µm respectively, the step portion 7 with the thickness of 350̸ µm is formed to by etching in order to set the thickness of the upper chamber to 50̸ µm. In this manner, when water is injected into the micro device 10̸ initially, the rate 50̸ of flow from the lower chamber 9 to the upper chamber 8 becomes higher than the conventional rate as shown in Fig. 3 so that a bubble 51 can be pushed out though the bubble intends to remain in the upper chamber 8 because the thickness of the upper chamber 8 is equal to the thickness of the lower chamber 9. Because the flow rate is proportional to the cube of the thickness of the upper chamber 8, the flow rate increases by 9 times when the thickness decreases to 1/3. Accordingly, the bubble can be escaped out more sufficiently. As a result, the bubble can be eliminated so that valve characteristic as expected can be maintained.

In this type micro device, the thickness of the silicon substrate 1 of not smaller than 220̸ µm, practically not smaller than 380̸ µm, is required to prevent cracking of the silicon substrate. Further, each of the thickness t1 of the lower chamber 9 and the thickness of the valve film 4 is selected to be not smaller than 50̸ µm on planning. Accordingly, in the case where a 50̸0̸ µm-thick silicon substrate is used in the conventional device, the difference difference between the two chambers 8 and 9 becomes very large as described previously, so that bubble escaping is poor due to shortage of the flow rate in the upper chamber 8 and the output passage 12. The difference between the thicknesses of the two chambers 8 and 9, however, can be eliminated by the provision of the step portion 7 as shown in this embodiment, so that the initial flow rate at the time of liquid injection can be increased to thereby improve bubble escaping characteristic. Further, according to an experiment, there has been found a result that the optimum value of the thickness of the upper chamber 8 is in a range of from 10̸ µm to 150̸ µm and that valve characteristic is lowered because it is difficult to eliminate the bubble if the thickness exceeds the range. Accordingly, it is preferable that the chamber thickness ratio t2/t1 is in a range of from 0̸.2 to 5.0̸.

### EMBODIMENT 2

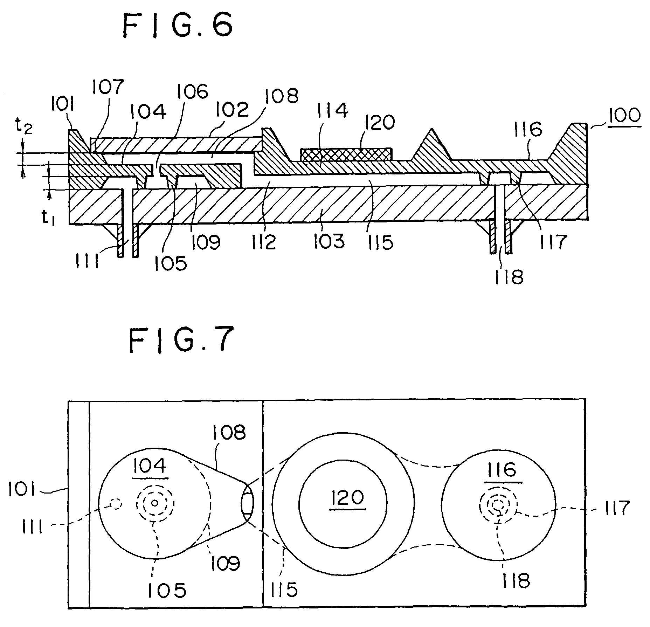

Fig. 4 is a sectional side view of a second embodiment of the present invention; and Fig. 5 is a sectional view taken along the line A-A in Fig. 4.

In this embodiment, the step portion 7 in the first embodiment is formed by etching the intermediate substrate 1 to make the height of the step portion 7 equal to the height of the valve film 4, and all parts of the upper chamber 8 are formed by etching on the upper substrate 2 side. The configuration of this embodiment except the above description is the same as the configuration of the first embodiment. The height of the step portion 7 can be determined arbitrarily. Alternatively, the chamber may be formed both in the upper substrate 2 and in the intermediate substrate 1 as long as the chamber thickness ratio t2/t1 satisfies the aforementioned range. Though not shown, the step portion may be provided on the lower chamber 9 side.

### EMBODIMENT 3

Next, an example of configuration of a micro pump as an example of application of the aforementioned micro device is shown in Fig. 6. Fig. 6 is a sectional side view of a micro pump; and Fig. 7 is a top view of the micro pump showing the case where the upper substrate of the micro pump is omitted. In the drawings, the reference numeral 10̸0̸ designates a micro pump; 10̸1, an intermediate silicon substrate; 10̸2, an upper glass substrate; and 10̸3, a lower glass substrate. Further, the reference numeral 10̸4 designates an input side valve film formed in the intermediate substrate 10̸1; 10̸5, an input valve portion; 10̸6, a vent; 10̸7, a step portion; 10̸8, an upper chamber; 10̸9, an lower chamber communicated with an input port 111; and 112, an output passage communicated with the output valve side through a pressure chamber 115 just under a diaphragm 114 to which a piezoelectric element 120̸ is mounted. Further, the reference numeral 116 designates an output side valve film formed in the intermediate substrate 10̸1; and 117, an output valve portion for opening/closing an output port 118 of the lower substrate 10̸3.

That is, this embodiment is formed by applying the configuration of the first embodiment to the input valve side so that the chamber thickness ratio t2/tl is set in a range of from 0̸.2 to 5.0̸ by the step portion 10̸7.

### EMBODIMENT 4

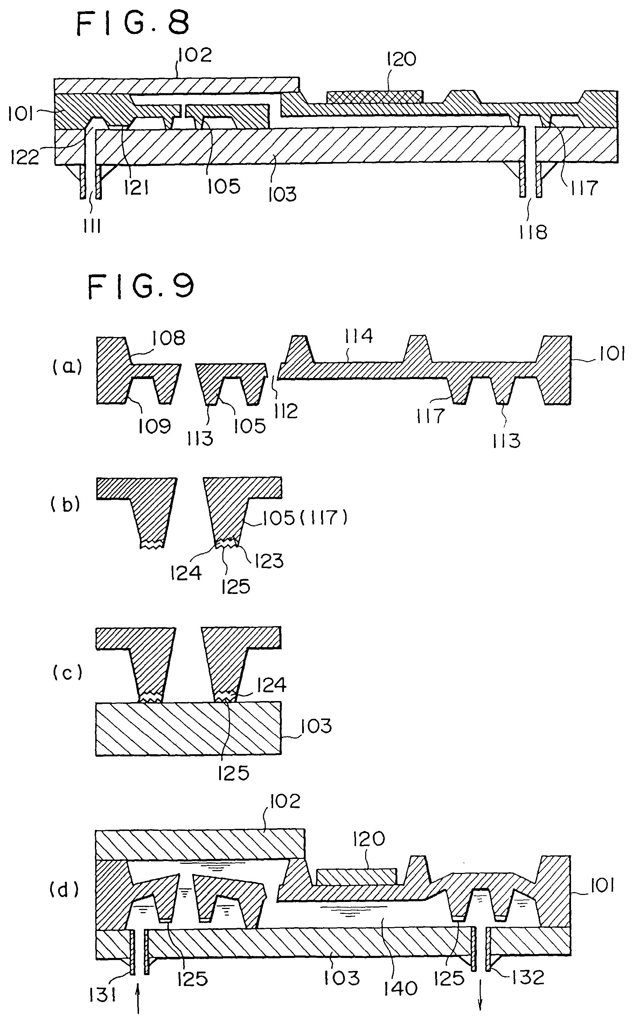

Fig. 8 is a sectional side view of a fourth embodiment. In this drawing, there is shown the case where the device is employed as a micro pump. The reference numeral 121 designates a comb filter portion provided to the input passage 122.

### EMBODIMENT 5

In the following, the structure for preventing valve sticking as one object of the present invention will be described in connection with the producing method.

Fig. 9 is a schematic production step view in the case of a micro pump, which view is expressed as a sectional view. Further, the diagrams (b) and (c) of Fig. 9 are views in which only the valve portion is enlarged.

The diagram (a) of Fig. 9 is a sectional side view of the intermediate silicon substrate 10̸1 as described above. Elements such as valve portions 10̸5 and 117, a diaphragm portion 114, flow passage portions 10̸8, 10̸9 and 112, etc. are formed in the intermediate substrate 10̸1 by etching a double-side-polished silicon substrate of crystal orientation (10̸0̸) with a solution of 30̸ % by weight of potassium hydroxide heated to 60̸°C from two surfaces while using a thermally oxidized film (silicon dioxide) as a patterning mask. A plurality of intermediate substrates 10̸1 each of which is a main part of the micro pump having the aforementioned structure are formed on a 4-inch silicon substrate.

With respect to the valve seal surface 113 of the intermediate substrate 10̸1, the state of a silicon polished surface generally remains without any change. If a micro pump is formed in this state, valve sticking occurs as described above.

In this embodiment, therefore, as shown in the diagram (b) of Fig. 9, a valve end surface 123 is first etched by 5 µm with the aforementioned potassium hydroxide solution to thereby set the roughness average of the surface to 10̸ nm. Then, the thermally oxidized film temporarily used as a mask is removed with a hydrofluoric acid solution. Then, thermal oxidation/ photolithography is applied onto the valve end portion 123 again to thereby prepare a 1.1 µm-thick thermally oxidized film 124 which is to be used as a pre-load.

After the valve seal surface 125 is formed as a rough surface in this manner, upper and lower substrates 10̸2 and 10̸3 made of Pyrex glass are joined like a sandwich by an anodic bonding method (320̸°C, 650̸ V). Then, the substrate is divided into individual micro pumps by dicing or the like. Finally, a driving piezoelectric element 120̸ is attached to the diaphragm portion 114, and liquid-in and -out pipes 131 and 132 are attached to the lower surface 10̸3 to thereby complete micro pumps (see the diagrams (c) and (d) in Fig. 9).

When liquid 140̸ is injected by using a micro pump thus prepared, the valve portions 10̸5 and 117 are opened easily by low pressure so that flowing-in of liquid and excretion of bubbles can be performed without any trouble (see the diagram (d) of Fig. 9).

### EMBODIMENT 6

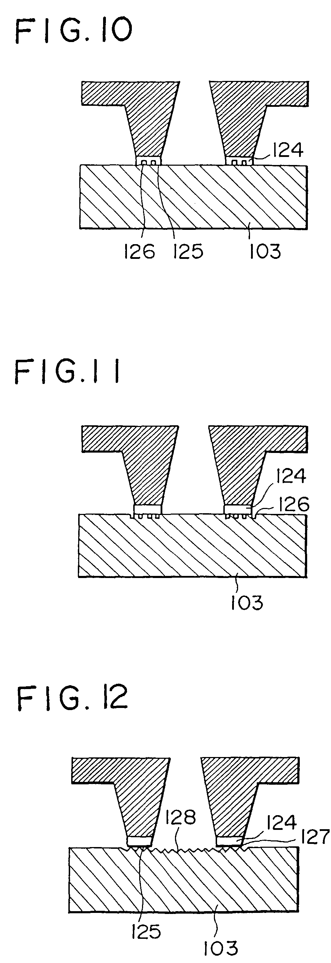

Fig. 10 is an enlarged sectional view of a valve portion of a micro pump according to a sixth embodiment.

The process for producing micro pumps is the same as in the ninth embodiment, except that the valve seal surface 125 in this embodiment is shaped to have a plurality of different level portions 126. That is, after the thermally oxidized film used as a mask for the valve end portion is removed with a hydrofluoric acid solution, thermal oxidation/photolithography is applied onto the valve end surface again to thereby prepare a 1.2 µm-thick thermally oxidized film 124 to be used as a pre-load. Then, after the pre-load portion 124 is patterned with OFPR-80̸0̸ posi-resist (produced by Tokyo Applied-Chemistry Co., Ltd.), etching with a solution of 8 % by weight of hydrofluoric acid is performed for 50̸ seconds to thereby prepare a 10̸0̸ nm different level shape. The plurality of step portions 126 are formed in the direction of the circumference of the valve seal surface 125.

Also in the micro pump of this embodiment, when liquid is injected, the liquid penetrates through the clearance of the different level portions 126 in the valve seal surface so that the valve portions can be opened easily by low pressure and, accordingly, excretion of bubbles is performed without any trouble.

### EMBODIMENT 7

Fig. 11 is an enlarged sectional view of a valve portion of a micro pump according to a seventh embodiment.

In this embodiment, different level portions 126 the same as in the sixth embodiment are formed in the upper surface portion of the lower substrate 10̸3 arranged opposite to the valve seal surface 125.

First, the valve seal surface 125 is prepared to a plane shape by forming a 1.3 µm-thick thermally oxidized film 124 to be used as a pre-load in the same manner as in the fifth and sixth embodiments. Then, before the lower substrate 10̸3 is anodically bonded with the intermediate substrate 10̸1, the upper surface portion of the lower substrate 10̸3 arranged opposite to the valve seal surface is patterned with OMR-85 negative resist (produced by Tokyo Applied-Chemistry Co., Ltd.) and then etching with a solution of 7.5 % by weight of hydrofluoric acid to thereby form the plurality of a 20̸0̸ nm different level portion 126 concentrically.

Also in the micro pump of this embodiment, the same function/effect as in the embodiment is attained.

### Embodiment 8

Fig. 12 is an enlarged sectional view of a valve portion of a micro pump according to an eighth embodiment.

In this embodiment, after a 1.2 µm-thick thermally oxidized film 124 as a pre-load layer as described above is formed, a 0̸.2 µm platina thin film 127 is formed thereon to thereby prepare the valve seal surface to a plane shape. Then, before anodic bonding the whole surface of the top surface portion of the lower substrate 10̸3 arranged opposite to the valve seal surface 125 is patterned with OMR-85 negative resist (produced by Tokyo Applied-Chemistry Co., Ltd.) and then etching with a solution of 7 % by weight of hydrofluoric acid is performed for 90̸ seconds to thereby form a rough surface 128 of average 10̸ nm.

As described above, valve sticking is prevented by forming either the valve seal surface or the top surface portion of the supporting substrate arranged opposite to the valve seal surface or both of them as a rough surface as long as leakage does not occur.

The lower limit of the surface roughness of the aforementioned rough surface is regarded as average 5 nm. This is because the surface is regarded as a good surface if the surface is not provided as a polished surface. This is based on the fact that the surface roughness of the polished surface is average 2 nm and maximum 22 nm.

Next, the upper limit of the surface roughness of the aforementioned rough surface may be 1 µm. It is experimentally confirmed that leakage does not occur even in the case of 1 µm.

### EMBODIMENT 9

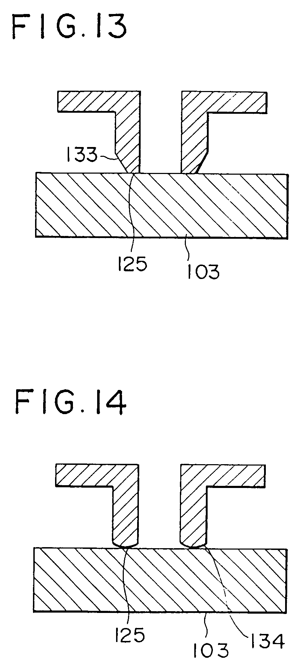

Further, the contact area of the valve seal surface 125 is set to be in a smaller value by applying tapering 133 or rounding 134 to the corners of the valve seal portion as shown in Figs. 13 and 14, so that the valve portions can be opened easily by pressure. Accordingly, there arises an effect on prevention of valve sticking.

### EMBODIMENT 10

In the following, the whole of the system using the micro pump according to the present invention will be described.

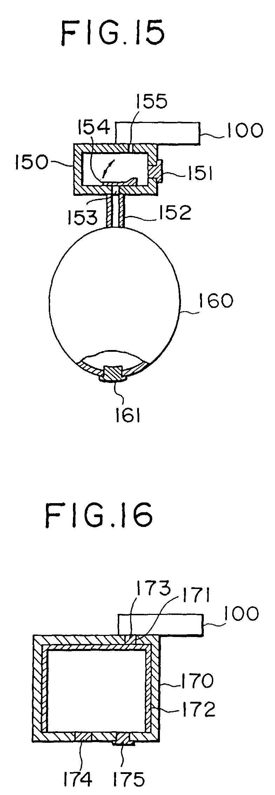

Fig. 15 is a schematic view in partly section showing the outline of a micro pump system according to a tenth embodiment.

This embodiment shows the case where a micro pump 10̸0̸ as shown in Figs. 6, 8, 9, etc. is used for medical treatment. The micro pump 10̸0̸ is connected to a large-scale soft tank 160̸ through a small-scaled tank 150̸.

The small-scaled tank 150̸ is a hard tank. A rubber stopper 151 called "septum" is fixed to the side wall of the small-scale tank 150̸ so as to be in a sealing condition. Further, a check valve 154 is mounted to a connection port 153 for connecting a pipe 152 to the large-scaled soft tank 160̸. The connection port 155 of the small-scaled tank 150̸ is connected to the aforementioned input port of the micro pump 10̸0̸.

Further, the large-scaled soft tank 160̸ is shaped like a transfusion pack. A septum 161 is mounted to the tank 160̸ in the same manner as described above. The output port of the micro pump 10̸0̸ is connected to an injection needle not shown.

A medical fluid is injected by thrusting the injection needle into the septum 151 of the small-scaled tank 150̸. The check valve 154 prevents the medical fluid injected into the small-scaled tank 150̸ from entering into the large-scaled soft tank 160̸, so that the internal pressure of the small-scaled tank 150̸ is not given to the large-scaled soft tank 160̸. Accordingly, the large-scaled soft tank 160̸ is not extended by the injected fluid. As a result, the medical fluid is injected into the micro pump 10̸0̸ through the connection port 155 in accordance with the injection pressure.

Next, the medical fluid can be reserved in the large-scaled soft tank 160̸ by thrusting the injection needle into the septum 161 thereof. Air remaining in the large-scaled soft tank 160̸ is escaped out by the injector through the septum 161 or entering of bubbles is inhibited by a filter.

Because the small-scaled hard tank 150̸ having a septum 151 and a check valve 154 is provided between the micro pump 10̸0̸ and the large-scaled soft tank 160̸ as described above, priming of the micro pump can be attained easily.

### EMBODIMENT 11

Fig. 16 is a schematic view of a micro pump according to an eleventh embodiment.

In the case of this system, it is assumed that the micro pump 10̸0̸ is used for general industry. The micro pump 10̸0̸ per se is the same as that in the tenth embodiment. This system is configured so that the hard tank 170̸ is connected to the micro pump 10̸0̸. Further, the aforementioned hydrophilic filters 172 and 173 are respectively provided to a part of the inner wall of the hard tank 170̸ and the connection port 171 between the hard tank and the micro pump 10̸0̸. Further, a hydrophobic filter 174 and a septum 175 are attached to the wall portion of the hard tank 170̸ in which there is no attachment of the hydrophilic filter. The "hydrophobic filter" means a filter which has properties reversed to the hydrophilic filter and which is impervious to water but pervious to air. The hydrophilic filter 173 provided in the connection port 171 is brought into close contact with the input port of the micro pump 10̸0̸.

A liquid is injected into the hard tank by thrusting the septum 175. The liquid in the inside is penetrated through the hydrophilic filters 172 and 173 in accordance with the injection pressure, so that the liquid is injected into the micro pump 10̸0̸ through the connection port 171. Entering of bubbles is inhibited by the hydrophilic filters 172 and 173, so that the bubbles do not enter into the micro pump 100. Accordingly, priming can be performed easily. Further, the hydrophobic filter 174 fulfills a respiratory function for the hard tank 170̸ at the time of operating of the micro pump 10̸0̸.

### EMBODIMENT 12

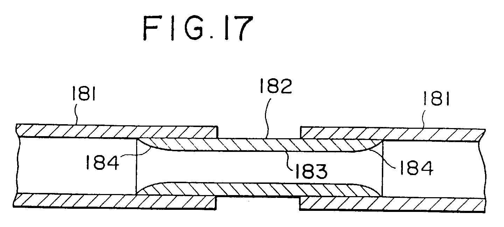

Fig. 17 shows an example of the structure of a relay pipe of pipes or tubes used for connecting micro pumps, tanks, etc. For example, pipes or tubes are connected to the input and output ports of the micro pump. A relay pipe having the structure in which the diameter of the inner hole of the relay pipe is made large by tapering or the like at each of the opposite ends of the relay pipe is used for the connection of these pipes or tubes. In the drawing, the reference numeral 181 designates pipes or tubes (hereinafter referred to as "pipe members"); and 182, a relay pipe which is forcedly inserted into the pipe members 181 to thereby connect the two pipe members. The opposite end portions of the inner hole 183 of the relay pipe 182 are tapered or rounded (hereinafter called "tapering or the like") to increase the diameter of the inner hole 183 up to its outer circumferential edge at each of the opposite end portions of the relay pipe 182.

It is general that the opposite end portions of the outer circumference of the relay pipe of this type are tapered to make connection of the pipe members 181 easy. When the opposite end portions of the outer circumference of the relay pipe are tapered, however, a gap is formed between the relay pipe and the pipe member 181 because of tapering. Bubbles in the injected liquid are deposited on the gap and, accordingly, the bubbles are separated to enter into the micro pump or the like.

In this embodiment, tapering or the like 184 is applied to the inner hole 184 at its opposite end portions so that the diameter of the inner hole 184 is increased up to the outer circumferential edge reversely to the conventional relay pipe. Accordingly, the aforementioned gap is not formed between the relay pipe 182 and the pipe member 181, so that bubbles in the injected liquid are hardly deposited. Accordingly, bubbles do not remain in the relay pipe portion of the pipe member 181, so that bubble escaping characteristic of the micro pump or the like is improved.

In the following, the producing method according to the present invention will be described in the case where the micro pump in Fig. 6 is used as an example.

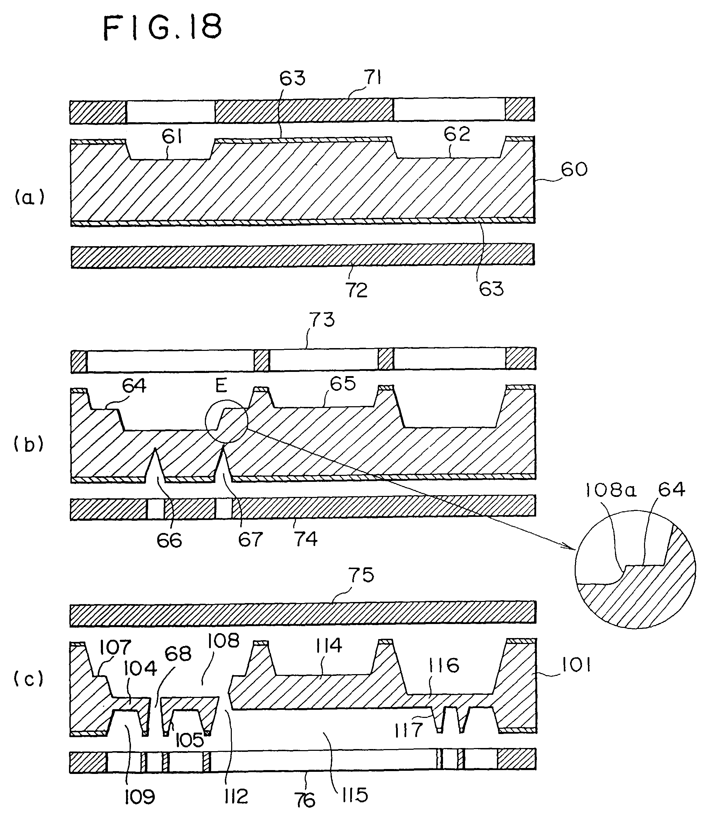

Fig. 18 is a schematic step view showing the step of etching a silicon substrate to form an intermediate substrate for the micro pump. Also referring to Fig. 6, description will be made.

This type etching is generally wet etching in which a pattern is formed by using photolithography.

As an etching solution, a KOH solution or a solution of EPW or hydrazine adapted to anisotropic etching is used as the etching solution. Further, in this example, silicon wafer of crystal orientation (10̸0̸) is used as the silicon substrate.

In the diagram (a) of Fig. 18, the reference numerals 71 and 72 designate photo masks which are patterned so as to form the input valve upper portion 61 and the output valve upper portion 62. In this case, positive resist is used. With respect to the etching step, a silicon substrate 60̸ with an oxidized film to which a resist is applied with the mask 71 as a pattern is exposed, developed and fixed. Then, windows are formed in the oxidized film 63 of the upper portion of the silicon substrate by a hydrofluoric acid solution. Then, the resist is separated so that etching with a KOH solution. Because silicon is anisotropically etched with KOH, the input valve upper portion 61 and the output valve upper portion 62 are formed in the silicon substrate 60̸ as shown in Fig. 18 (a).

Then, as shown in the diagram (b) of Fig. 18, the silicon substrate 60̸ is etched with the aforementioned KOH solution while using a mask 73 to form the step portion 64 and the diaphragm upper portion 65. In the case, the step portion 64 and the diaphragm upper portion 65 are formed simultaneously. Further, a mask 74 to form V grooves 66 and 67 is used so that the through hole of the input valve portion and the passage portion of the silicon substrate lower portion are formed in the next etching step. Because the input valve upper portion 61 is not masked here, etching in the (111) surface cannot be stopped. Accordingly, because side etching is made, it is necessary that the quantity of side etching is calculated in advance.

Then, in the diagram (c) of Fig. 18, a mask 75 is provided for protecting the not-etched portion in the upper portion of the silicon substrate. Accordingly, the mask 75 may be black entirely. Then, the lower portion of the silicon substrate is etched while using a mask 76 to form the passage portion and the valve portion in the lower portion of the silicon substrate. Because the V groove 66 formed in the diagram (b) of Fig. 18 is not masked here, the V groove is side-etched to form a through hole 68 (10̸6 in Fig. 6). Then, an intermediate substrate in which respective parts such as valve films 10̸4 and 116, valve portions 10̸5 and 117, a lower chamber 10̸9, an upper chamber 10̸8, a step portion 10̸7, an output passage 112, a diaphragm portion 114 and a pressure chamber 115 are formed as shown in Fig. 6 can be obtained. Because the height of the upper surface of the diaphragm portion 114 and the height of the upper surface of the step portion 10̸7 are equal to each other, the thickness of the upper chamber 10̸8 is univocally determined on the basis of the difference between the thickness of the diaphragm portion 114 and the thickness of the input valve film 10̸4. When, for example, the thickness of the input valve film 10̸4 and the thickness of the diaphragm portion 114 are 40̸ µm and 60̸ µm respectively, the thickness of the upper chamber 10̸8 is determined to 20̸ µm. Though not obvious in the diagram (b) of Fig. 18, the side wall 10̸8a of the upper chamber 10̸8 is smoothened as shown in the enlarged view of D portion in the same diagram by etching the whole of the input valve upper portion 61. Accordingly, there is obtained an effect that bubble escaping characteristic is improved.

## Claims

1. A fluid control micro device comprising three substrates joined in a multi-layer structure by an anodic joining method, said three substrates being an upper substrate (2, 102), an intermediate substrate (1, 101) and a lower substrate (3, 103), said intermediate substrate (1, 101) comprising a valve film (4, 104) which separates an input side space formed between said upper and lower substrates into an upper chamber (8, 108) and a lower chamber (9,109), said valve film having a cylindrical valve portion (5, 105) for communicating or blocking said upper chamber (8, 108) and said lower chamber (9, 109) in accordance with upward and downward transformation of said valve film (4, 104), wherein the ratio of the thickness t2 of said upper chamber (8, 108) to the thickness tl of said lower chamber (9, 109) is selected to be in the range t2/t1 = 0.2 to 5.0, characterized in that said upper substrate (2, 102) and said intermediate substrate (1, 101) are joined at a step portion (7, 107) to reduce the thickness of said upper chamber (8, 108).

2. A fluid control micro device according to Claim 1, characterized in that said step portion (7, 107) is formed in said intermediate substrate (1, 101), said upper substrate (2, 102), or in both said intermediate and said upper substrates.

3. A fluid control micro device according to Claim 1, characterized in that the joined portion of said intermediate substrate (1, 101) and said lower substrate (3, 103) has a step portion so that the thickness of said lower chamber (9, 109) can be reduced.

4. Preferably, the fluid control micro device of the present invention is further characterized in that said intermediate substrate has a pressure chamber communicating with an output passage of a flow passage system, a diaphragm portion above said pressure chamber, a piezo-electric element for driving said diaphragm portion, and an output valve portion communicating with said pressure chamber for opening/closing an output port, said fluid control micro device constituting a micro pump.

5. A fluid control micro device according to Claim 4, characterized in that said flow passage system in said intermediate substrate is provided at the side of said upper substrate (2, 102).

6. A fluid control micro device according to Claim 1 or Claim 4, characterized in that a filter portion constituted by at least one groove is provided in an input passage (14, (111) in said flow passage system (14, 6, 12; 111, 106, 112).

7. A fluid control micro device according to Claim 6, characterized in that said filter portion is formed like a comb in said intermediate substrate (1, 101).

8. A fluid control micro device according to Claim 6, characterized in that said filter portion is formed like a comb in said lower substrate (3, 103).

9. A fluid control micro device according to Claim 6, characterized in that said filter portion is constituted by narrow gaps between a projection portion provided on said intermediate substrate (1, 101) and said lower substrate (3, 103).

10. A fluid control micro device according to Claim 4, characterized in that a hydrophilic filter is attached directly to a portion of input port (11, 111) provided in said lower substrate (3, 103).

11. A fluid control micro device according to Claim 10, characterized in that said filter is constituted by a combination of a laminate of a plurality of filters different in pore size from each other, and a hydrophilic filter.

12. A fluid control micro device according to Claim 1 or Claim 4, characterized in that at least one of a seal surface (125) of said valve portion (5, 105) and a surface portion of said lower substrate (3, 103) opposite to said valve portion is formed as a rough surface (128) having a surface roughness in the range from 5 nm to 1000 nm as average.

13. A fluid control micro device according to Claim 12, characterized in that said rough surface (128) is formed as a plurality of grooves (126) provided on the lower surface of said valve portion (5, 105) or on the upper surface of said lower substrate (3, 103).

14. A fluid control micro device according to Claim 13, characterized in that said plurality of grooves (126) is formed circumferentially in said seal surface (125) of the valve portion (5, 105).

15. A fluid control micro device according to Claim 13, characterized in that said plurality of grooves (126) is formed concentrically in the surface portion of said lower substrate (3, 103) opposite to said valve portion (5, 105).

16. A fluid control micro device according to Claim 13, characterized in that said seal surface (125) of the valve portion (5, 105), is formed by a pre-load layer of a thermally oxidized film (124).

17. A fluid control micro device according to Claim 13, characterized in that said seal surface (125) of the valve portion (5, 105) is formed as a platina thin film layer (127) formed on a pre-load layer of a thermally oxidized film (124).

18. A control fluid micro device according to Claim 1 or Claim 4, characterized in that a control area of said seal portion (125) of the valve portion (5, 105) is reduced by tapering or rounding said seal portion.

19. A fluid control micro device according to Claim 1 or Claim 4, characterized in that said device has a small-scaled hard tank (150) connected to an input port (11, 111) of said flow passage system, and a large-scaled soft tank (160) connected to said small-scaled hard tank (150), said small-scaled hard tank having a septum (151) attached to the wall thereof, and a check valve (154) for inhibiting flow of a fluid into said large-scaled soft tank (160).

20. A fluid control micro device according to Claim 19, characterized in that a laminate of a plurality of filters different in pore size from each other, and a hydrophilic filter, are attached to a portion of an input port (11, 111) of said flow passage system.

21. A fluid control micro device according to Claim 1 or Claim 4, characterized in that said device has a hard tank (170) connected to an input port (11, 111) of said flow passage system, said hard tank (170) having a hydrophilic filter (173) attached to the inside of the connection port (171) thereof, a hydrophilic filter (172) covering most of the inside surface of said hard tank (170) except one portion, and a hydrophobic filter (174) and a septum (175) attached to the uncovered wall portion of said hard tank (170).

22. A fluid control micro device according to Claim 1 or Claim 4, characterized in that said device has relay pipe members (181) connected to an input port (11, 111) of said flow passage system, said relay pipe members (181) having a relay pipe (182) formed to have a tapered or rounded shape (184) in which the inner surface of said relay pipe (181) is tapered or rounded so that the diameter of the inner hole (183) of said relay pipe (181) increases to the outer circumferential edge of each of the opposite end portions of said relay pipe (181).

23. A method for producing a fluid control micro device comprising joining in a multilayer structure by means of an anodic joining method, three substrates consisting of an upper substrate (2, 102), an intermediate substrate (1, 101) and a lower substrate (3, 103), characterised in that said intermediate substrate (1, 101) is anisotropically etched using a pattern mask (73) thereby to simultaneously form on an input side upper chamber (108) a step portion (107) equal in height to a diaphragm portion (114).

## Patentansprüche

1. Microfluidsteuervorrichtung mit drei Substraten, die in einer Mehrschichtanordnung durch ein anodisches Verbindungsverfahren verbunden sind, wobei die drei Substrate ein oberes Substrat (2,102), ein mittleres Substrat (1,101) und ein unteres Substrat (3,103) sind, das mittlere Substrat (1,101) einen Ventilfilm (4,104) aufweist, welcher einen einlaßseitigen Raum, der zwischen dem oberen und unteren Substrat gebildet ist, in eine obere Kammer (8,108) und eine untere Kammer (9,109) trennt, wobei der Ventilfilm einen zylindrischen Ventilteil (5,105) zum Verbinden oder Sperren der oberen Kammer (8,108) bezüglich der unteren Kammer (9,109) entsprechend der Aufwärts- und Abwärtsverformung des Ventilfilms (4,104) aufweist, und wobei das Verhältnis der Dicke t2 der oberen Kammer (8,108) zur Dicke tl der unteren Kammer (9,109) so gewählt ist, daß es im Bereich t2/t1 = 0,2 bis 5,0 liegt, dadurch gekennzeichnet, daß das obere Substrat (2,102) und das mittlere Substrat (1,101) durch einen Stufenteil (7,107) verbunden sind, um die Dicke der oberen Kammer (8,108) zu verringern.

2. Microfluidsteuervorrichtung nach Anspruch 1, dadurch gekennzeichnet, daß der Stufenteil (7,107) in dem mittleren Substrat (1,101), dem oberen Substrat (2,102) oder sowohl im mittleren als auch oberen Substrat ausgebildet ist.

3. Microfluidsteuervorrichtung nach Anspruch 1, dadurch gekennzeichnet, daß der verbundene Teil des mittleren Substrats (1,101) und des unteren Substrats (3,103) einen Stufenteil aufweist, so daß die Dicke der unteren Kammer (9,109) verringert werden kann.

4. Microfluidsteuervorrichtung nach Anspruch 1, dadurch gekennzeichnet, daß das mittlere Substrat (101) eine Druckkammer (115), die mit einem Auslaßkanal (112) eines Strömungskanalsystems (111,106,112) in Verbindung steht, einen Membranteil (114) oberhalb der Druckkammer (115), ein piezoelektrisches Element (120) zum Antreiben des Membranteils (114) und einen Auslaßventilteil (117) aufweist, der mit der Druckkammer (115) zum Öffnen/Schließen einer Auslaßöffnoung (118) in Verbindung steht, wobei die Microfluidsteuervorrichtung eine Micropumpe bildet.

5. Microfluidsteuervorrichtung nach Anspruch 4, dadurch gekennzeichnet, daß das Strömungskanalsystem in dem mittleren Substrat auf der Seite des oberen Substrats (2,102) vorgesehen ist.

6. Microfluidsteuervorrichtung nach Anspruch 1 oder 4, dadurch gekennzeichnet, daß ein Filterteil , der von wenigstens einer Nut gebildet wird, in einem Einlaßkanal (14,111) in dem Strömungskanalsystem (14,6,12; 111,106, 112) vorgesehen ist.

7. Microfluidsteuervorrichtung nach Anspruch 6, dadurch gekennzeichnet, daß der Filterteil in dem mittleren Substrat (1,101) wie ein Kamm ausgebildet ist.

8. Microfluidsteurvorrichtung nach Anspruch 6, dadurch gekennzeichnet, daß der Filterteil in dem unteren Substrat (3,103) wie ein Kamm ausgebildet ist.

9. Microfluidsteuervorrichtung nach Anspruch 6, dadurch gekennzeichnet, daß der Filterteil aus schmalen Spalten zwischen einem vorspringenden Teil besteht, die am mittleren Substrat (1,101) und dem unteren Substrat (3,103) vorgesehen sind.

10. Microfluidsteuervorrichtung nach Anspruch 4, dadurch gekennzeichnet, daß ein hydrophiles Filter direkt an einem Teil des Einlaßkanals (11,111) befestigt ist, der in dem unteren Substrat (3,103) vorgesehen ist.

11. Microfluidsteuervorrichtung nach Anspruch 10, dadurch gekennzeichnet, daß das Filter aus einer Kombination eines Laminats mehrerer Filter , deren Porengröße jeweils unterschiedlich ist, und einem hydrophilen Filter besteht.

12. Microfluidsteuervorrichtung nach Anspruch 1 oder 4, dadurch gekennzeichnet, daß wenigstens eine Dichtungsfläche (125) des Ventilteils (5,105) oder ein Oberflächenteil des unteren Substrats (3,103) gegenüber dem Ventilteil als rauhe Oberfläche (128) mit einer Oberflächenrauhheit im Bereich von 5 nm bis 100 nm im Durchschnitt ausgebildet ist.

13. Microfluidsteuervorrichtung nach Anspruch 12, dadurch gekennzeichnet, daß die rauhe Oberfläche (128) als Mehrzahl von Nuten (126) ausgebildet ist, die auf der unteren Fläche des Ventilteils (5,105) oder auf der oberen Fläche des unteren Substrats (3,103) vorgesehen sind.

14. Microfluidsteuervorrichtung nach Anspruch 13, dadurch gekennzeichnet, daß die Mehrzahl von Nuten (126) in Umfangsrichtung der Dichtungsfläche (125) des Ventilteils (5,105) ausgebildet ist.

15. Microfluidsteuervorrichtung nach Anspruch 13, dadurch gekennzeichnet, daß die Mehrzahl von Nuten (126) konzentrisch im Oberflächenteil des unteren Substrats (3,103) gegenüber dem Ventilteil (5,105) ausgebildet ist.

16. Microfluidsteuervorrichtung nach Anspruch 13, dadurch gekennzeichnet, daß die Dichtungsfläche (125) des Ventilteils (5,105) von einer Vorbelastungsschicht eines thermisch oxidierten Films (124) gebildet ist.

17. Microfluidsteuervorrichtung nach Anspruch 13, dadurch gekennzeichnet, daß die Dichtungsfläche (125) des Ventilteils (5,105) aus einer dünnen Platin-Filmschicht (127) gebildet ist, die auf einer Vorbelastungsschicht eines thermisch oxidierten Films (124) ausgebildet ist.

18. Microfluidsteuervorrichtung nach Anspruch 1 oder 4, dadurch gekennzeichnet, daß ein Steuerbereich des Dichtungsteils (125) des Ventilteils (5,105) durch Abschrägen oder Abrunden des Dichtungsteils reduziert ist.

19. Microfluidsteuervorrichtung nach Anspruch 1 oder 4, dadurch gekennzeichnet, daß die Vorrichtung einen kleinen harten Behälter (150), der mit einer Einlaßöffnung (11,111) des Strömungskanalsystems verbunden ist, und einen großen weichen Behälter (160) aufweist, der mit dem kleinen harten Behälter (150) verbunden ist, wobei der kleine harte Behälter eine an seiner Wand befestigte Trennwand (151) und ein Rückschlagventil (154) zur Verhinderung einer Fluidströmung in den großen weichen Behälter (160) aufweist.

20. Microfluidsteuervorrichtung nach Anspruch 19, dadurch gekennzeichnet, daß ein Laminat aus einer Mehrzahl von Filtern , deren Porengröße jeweils unterschiedlich ist, und ein hydrophiles Filter an einem Teil einer Einlaßöffnung (11,111) des Strömungskanalsystems befestigt ist.

21. Microfluidsteuervorrichtung nach Anspruch 1 oder 4, dadurch gekennzeichnet, daß die Vorrichtung einen harten Behälter (170) aufweist, der mit einer Einlaßöffnung (11,111) des Strömungskanalsystems verbunden ist, wobei der harte Behälter (170) ein hydrophiles Filter (173), das an der Innenseite seiner Anschlußöffnung (171) befestigt ist, ein hydrophiles Filter (172), das den größten Teil der Innenfläche des harten Behälters (170) mit Ausnahme eines Teils bedeckt, und ein hydrophobes Filter (174) sowie eine Trennwand (175) aufweist, die am unbedeckten Wandteil des harten Behälters (170) befestigt sind.

22. Microfluidsteuervorrichtung nach Anspruch 1 oder 4, dadurch gekennzeichnet, daß die Vorrichtung Übertragungsrohrorgane (181) aufweist, die mit einer Einlaßöffnung (11,111) des Strömungskanalsystems verbunden sind, wobei die Übertragungsrohrorgane (181) ein Übertragungsrohr (182) aufweisen, das so ausgebildet ist, daß es eine abgeschrägte oder abgerundete Form (184) besitzt, bei welcher die Innenfläche des Übertragungsrohrs (181) derart abgeschrägt oder abgerundet ist, daß der Durchmesser der Innenöffnung (183) des Übertragungsrohrs (181) zum äußeren Umfangsrand jedes der entgegengesetzten Endabschnitte des Übertragungsrohrs (181) zunimmt.

23. Verfahren zur Herstellung einer Microfluidsteuervorrichtung, bei welchem in einer Mehrschichtanordnung mittels eines anodischen Verbindungsverfahrens drei Substrate verbunden werden, die aus einem oberen Substrat (2,102) einem mittleren Substrat (1,101) und einem unteren Substrat (3,103) bestehen, dadurch gekennzeichnet, daß das mittlere Substrat (1,101) unter Verwendung einer Maskierungsschablone (73) anisotrop geätzt und dadurch gleichzeitig an einer auf der Einlaßseite gelegenen oberen Kammer (108) ein Stufenteil (107) ausgebildet wird, dessen Höhe derjenigen eines Membranteils (114) entspricht.

## Revendications

1. Microdispositif de commande du débit d'un fluide, comportant trois substrats liés dans une structure multicouche par un procédé de liaison anodique, lesdits trois substrats étant un substrat supérieur (2, 102), un substrat intermédiaire (1, 101) et un substrat inférieur (3, 103), ledit substrat intermédiaire (1, 101) comprenant un film formant vanne (4, 104) qui sépare un espace latéral d'entrée formé, entre lesdits substrats supérieur et inférieur, en une chambre supérieure (8, 108) et une chambre inférieure (9, 109), ledit film formant vanne ayant une portion cylindrique formant vanne (5, 105) pour faire ou non communiquer ladite chambre supérieure (8, 108) et ladite chambre inférieure (9, 109) en accord avec une transformation, vers le haut et vers le bas, dudit film formant vanne (4, 104), dispositif dans lequel le rapport entre l'épaisseur t2 de ladite chambre supérieure (8, 108) et l'épaisseur tl de ladite chambre inférieure (9, 109) est choisi pour être sur la plage t2/t1 = 0,2 à 5,0, caractérisé par le fait que ledit substrat supérieur (2, 102) et ledit substrat intermédiaire (1, 101) sont liés sur une portion en échelon (7, 107) pour réduire l'épaisseur de ladite chambre supérieure (8, 108).

2. Microdispositif de commande du débit d'un fluide selon la revendication 1, caractérisé par le fait que ladite portion en échelon (7, 107) est formé dans ledit substrat intermédiaire (1, 101), dans ledit substrat supérieur (2, 102), ou à la fois dans ledit substrat intermédiaire et dans ledit substrat supérieur.

3. Microdispositif de commande du débit d'un fluide selon la revendication 1, caractérisé par le fait que la portion liée dudit substrat intermédiaire (1, 101) et dudit substrat inférieur (3, 103) présente une portion en échelon de façon à pouvoir réduire l'épaisseur de ladite chambre inférieure (9, 109).

4. Microdispositif de commande du débit d'un fluide selon la revendication 1, caractérisé par le fait que ledit substrat intermédiaire (101) comporte une chambre sous pression (115) qui communique avec un passage de sortie (112) d'un système (111, 106, 112) de passage pour écoulement, une portion formant membrane (114) au-dessus de ladite chambre sous pression (115), un élément piézoélectique (120) pour entraîner ladite portion formant membrane (114) et une portion (117) formant vanne de sortie communiquant avec ladite chambre sous pression (115) pour ouvrir/fermer un orifice de sortie (118), ledit microdispositif de commande du débit d'un fluide constituant une micropompe.

5. Microdispositif de commande du débit d'un Fluide selon la revendication 4, caractérisé par le fait que ledit système de passage pour écoulement est prévu, dans ledit substrat intermédiaire, du côté dudit substrat supérieur (2, 102).

6. Microdispositif de commande du débit d'un fluide selon la revendication 1 ou la revendication 4, caractérisé par le fait qu'une portion formant filtre, constituée par au moins une rainure, est prévue dans un passage d'entrée (14, 111) dans ledit système de passage pour écoulement (14, 6, 12; 111, 106, 112).

7. Microdispositif de commande du débit d'un fluide selon la revendication 6, caractérisé par le fait que ladite portion formant filtre est formée, en forme de nid d'abeilles, dans ledit substrat intermédiaire (1, 101).

8. Microdispositif de commande du débit d'un fluide selon la revendication 6, caractérisé par le fait que ladite portion formant filtre est formée, en forme de nid d'abeilles, dans ledit substrat inférieur (3, 103).

9. Microdispositif de commande du débit d'un fluide selon la revendication 6, caractérisé par le fait que ladite portion formant filtre est constituée par des interstices étroits entre une portion saillante prévue sur ledit substrat intermédiaire (1, 101) et ledit substrat inférieur (3, 103).

10. Microdispositif de commande du débit d'un fluide selon la revendication 4, caractérisé par le fait qu'un filtre hydrophile est directement fixé à une portion de l'orifice d'entrée (11, 111) prévu dans ledit substrat inférieur (3, 103).

11. Microdispositif de commande du débit d'un fluide selon la revendication 10, caractérisé par le fait que ledit filtre est constitué par une combinaison d'un empilement d'une pluralité de filtres différents l'un de l'autre en dimension de pores, et d'un filtre hydrophile.

12. Microdispositif de commande du débit d'un fluide selon la revendication 1 ou la revendication 4, caractérisé par le fait qu'au moins l'une, de la surface d'obturation (125) de ladite portion formant vanne (5, 105) et de la portion de surface dudit substrat inférieur (3, 103) située en face de ladite portion formant vanne, est formée en surface rugueuse (128) ayant une rugosité de surface sur la plage allant de 5 nm à 1000 nm en moyenne.

13. Microdispositif de commande du débit d'un fluide selon la revendication 12, caractérisé par le fait que ladite surface rugueuse (128) est formée comme une pluralité de rainures (126) prévues sur la surface inférieure de ladite portion formant vanne (5, 105) ou sur la surface supérieure dudit substrat inférieur (3, 103).

14. Microdispositif de commande du débit d'un fluide selon la revendication 13, caractérisé par le fait que ladite pluralité de rainures (126) a la forme de circonférences dans ladite surface d'obturation (125) de la portion formant vanne (5, 105).

15. Microdispositif de commande du débit d'un fluide selon la revendication 13, caractérisé par le fait que ladite pluralité de rainures (126) sont de forme concentrique dans la portion de surface dudit substrat inférieur (3, 103) située en face de ladite portion formant vanne (5, 105).

16. Microdispositif de commande du débit d'un fluide selon la revendication 13, caractérisé par le fait que ladite surface d'obturation (125) de la portion formant vanne (5, 105) est formée par une couche de précharge d'un film oxydé thermiquement (124).

17. Microdispositif de commande du débit d'un fluide selon la revendication 13, caractérisé par le fait que ladite surface d'obturation (125) de la portion formant vanne (5, 105) a la forme d'une couche de fin film de platine (127) formée sur une couche de précharge d'un film oxydé thermiquement (124).

18. Microdispositif de commande du débit d'un fluide selon la revendication 1 ou la revendication 4, caractérisé par le fait que l'on réduit une zone de commande de ladite portion d'obturation (125) de la portion formant vanne (5, 105) en amincissant ou en arrondissant ladite portion d'obturation.

19. Microdispositif de commande du débit d'un fluide selon la revendication 1 ou la revendication 4, caractérisé par le fait que ledit dispositif comporte un réservoir rigide (150), de petite dimension, relié à un orifice d'entrée (11, 111) dudit système de passage pour écoulement, et un réservoir souple (160), de grande dimension, relié audit réservoir rigide (150) de petite dimension, ledit réservoir rigide de petite dimension comprenant un bouchon de caoutchouc (151) fixé à sa paroi et un clapet de non-retour (154) pour interdire l'écoulement d'un fluide dans ledit réservoir souple de grande dimension (160).

20. Microdispositif de commande du débit d'un fluide selon la revendication 19, caractérisé par le fait qu'un empilement d'une pluralité de filtres, de dimension de pores différente l'un de l'autre, ainsi qu'un filtre hydrophile, sont fixés à une portion d'un orifice d'entrée (11, 111) dudit système de passage pour écoulement.

21. Microdispositif de commande du débit d'un fluide selon la revendication 1 ou la revendication 4, caractérisé par le fait que ledit dispositif comporte un réservoir rigide (170) connecté à un orifice d'entrée (11, 111) dudit système de passage pour écoulement, ledit réservoir rigide (70) ayant un filtre hydrophile (513) attaché à l'intérieur de son orifice de connexion (171), un filtre hydrophile (172) recouvrant la plus grande partie de la surface intérieure dudit réservoir rigide (170) à l'exception d'une portion et un filtre hydrophobe (174) et un bouchon de caoutchouc (175) attachés à la portion de paroi non recouverte dudit réservoir rigide (170).

22. Microdispositif de commande du débit d'un fluide selon la revendication 1 ou la revendication 4, caractérisé par le fait que ledit dispositif comporte des éléments (181) à conduite relais connectés à un orifice d'entrée (11, 111) dudit système de passage pour écoulement, lesdits éléments à conduite relais (181) ayant une conduite relais (182) formée pour avoir une forme amincie ou arrondie (184), dans lesquels c'est la surface intérieure de ladite conduite relais (181) qui est amincie ou arrondie de façon que le diamètre du trou intérieur (183) de ladite conduite relais (181) croisse dans la direction du bord circonférentiel extérieur de chacune des portions d'extrémité opposées de ladite conduite relais (181).

23. Procédé de production d'un microdispositif de commande du débit d'un fluide consistant à lier, dans une structure multicouche, au moyen d'un procédé de liaison anodique, trois substrats constitués d'un substrat supérieur (2, 102), d'un substrat intermédiaire (1, 101) et d'un substrat inférieur (3, 103), caractérisé par le fait que l'on attaque par voie anisotropique ledit substrat intermédiaire (1, 101) en utilisant un masque de motif (73), formant ainsi simultanément, sur une chambre supérieure (108) située du côté entrée, une portion en échelon (107) de hauteur égale à la portion formant membrane (114).
